(19) Europäisches Patentamt
European Patent Office
Office européen des brevets

(11) **EP 0 745 410 B1**

(12) **FASCICULE DE BREVET EUROPEEN**

(45) Date de publication et mention
de la délivrance du brevet:
**04.09.2002 Bulletin 2002/36**

(51) Int Cl.$^7$: **A61N 1/365**

(21) Numéro de dépôt: **96401162.1**

(22) Date de dépôt: **30.05.1996**

(54) **Dispositif médical implantable actif, notamment stimulateur cardiaque ou défribrillateur**

Aktive implantierbare medizinische Vorrichtung, insbesondere Herzschrittmacher oder Defibrillator

Active implantable medical device, especially pacemaker or defibrillator

(84) Etats contractants désignés:
**BE CH DE FR IT LI SE**

(30) Priorité: **31.05.1995 FR 9506476**

(43) Date de publication de la demande:
**04.12.1996 Bulletin 1996/49**

(73) Titulaire: **ELA MEDICAL (Société anonyme)**
**F-92541 Montrouge (FR)**

(72) Inventeurs:
• **Legay, Thierry**
**91640 Fontenay les Briis (FR)**

• **Bonnet, Jean-Luc**
**92170 Vanves (FR)**
• **Geroux, Laurence**
**92350 Le Plessis Robinson (FR)**

(74) Mandataire: **Dupuis-Latour, Dominique et al**
**Avocat à la Cour,**
**Cabinet Bardehle, Pagenberg & Partner,**
**14 boulevard Malesherbes**
**75008 Paris (FR)**

(56) Documents cités:
**EP-A- 0 488 841          EP-A- 0 647 454**
**WO-A-92/03182          WO-A-93/16756**
**US-A- 3 693 626          US-A- 5 330 513**

**Description**

La présente invention concerne les "dispositifs médicaux implantables actifs" tels que définis par la Directive 90/385/CEE du 20 juin 1990 du Conseil des Communautés Européennes, et plus précisément les stimulateurs cardiaques ou défibrillateurs, dont la fréquence de stimulation peut évoluer au-dessus d'une fréquence de base programmée.

Les premiers dispositifs de ce type étaient les stimulateurs mono-chambre "sentinelle" disposant d'un algorithme de lissage permettant de suivre le rythme spontané au-dessus du rythme de base, en assurant, en cas de disparition de ce rythme spontané, une continuité dans le rythme de délivrance des impulsions de stimulation en produisant une fréquence de valeur progressivement décroissante jusqu'à rejoindre la fréquence de base.

D'autres dispositifs de ce type sont les stimulateurs double chambre à synchronisation auriculo-ventriculaire, dans lesquels le ventricule est, normalement, stimulé au rythme de l'oreillette. En cas de trouble auriculaire, le ventricule est découplé de l'oreillette et la fréquence de stimulation doit, ici encore, décroître progressivement jusqu'à rejoindre une fréquence de consigne.

De la même façon, dans les dispositifs les plus récents où la fréquence de stimulation est asservie à l'information délivrée par un ou plusieurs capteurs, en cas de disparition de l'information capteur, ou de diminution trop brutale de cette dernière, le rythme de stimulation doit décroître jusqu'à une fréquence de consigne sans suivre les informations du ou des capteurs, de manière à respecter la physiologie du patient.

Les EP-A-0 488 840 (Ela Médical) et EP-A-0 488 841 (Ela Médical) enseignent diverses manières de contrôler la décroissance du rythme cardiaque dans ces diverses configurations de fonctionnement, par exemple au moyen de pentes de décélération (définies en millisecondes par cycle cardiaque) qui sont préprogrammées et mises en oeuvre lorsque l'appareil décide qu'il y a lieu de provoquer un retour à la fréquence de consigne.

Le paramètre de pilotage de la décroissance du rythme cardiaque peut être également défini sur la base d'un temps de descente entre le moment du déclenchement de la décroissance et l'instant final où le rythme programmé de base doit être atteint.

Le WO-A-93/23115 (Medtronic) propose, quant à lui, un algorithme de décroissance dans lequel les pentes de décélération sont calculées à partir d'un paramètre recueilli par un capteur physiologique, supposé délivrer une information de "travail" correspondant à l'activité du patient.

Il en est de même pour les dispositifs décrits par les WO-A-92/03182 (Medtronic) et EP-A-0 647 454 (Vitatron Medical).

Ce procédé de gestion produit cependant une diminution du rythme cardiaque qui n'est corrélée qu'aux informations issues du capteur (ce qui présuppose en outre que le stimulateur est du type asservi à un paramètre physiologique et dispose nécessairement d'un ou plusieurs capteurs de mesure).

L'un des buts de l'invention est de proposer une solution qui s'affranchisse de l'information issue du capteur, grâce à un stimulateur cardiaque ou défibrillateur implantable susceptible de faire décroître la fréquence stimulée d'une façon la mieux adaptée au patient en fonction de son rythme cardiaque antérieur, donc en respectant ses besoins physiologiques.

En d'autres termes, en cas d'effort peu important, le système pilotera une diminution rapide de la fréquence et, au contraire, allongera le temps de descente par une décroissance moins rapide en cas d'effort violent et/ou prolongé, de manière à ménager au patient un temps de récupération suffisant, adapté à ses besoins métaboliques réels.

On verra également que l'invention peut être mise en oeuvre dans tout type de stimulateur, que son fonctionnement soit ou non asservi à un capteur. L'invention peut donc être appliquée à une gamme complète de stimulateurs, y compris les plus simples, car elle ne nécessite pas le recueil d'informations externes, à l'exception du rythme cardiaque.

Le stimulateur de la présente invention comporte à cet effet les caractéristiques énoncées dans la revendication 1.

Les sous-revendications visent des mises en oeuvre avantageuses de l'invention

D'autres caractéristiques et avantages de l'invention apparaîtront à la lecture de la description détaillée ci-dessous, faite en référence aux dessins annexés.

La figure 1 est un schéma par blocs d'un stimulateur, asservi ou non, susceptible de mettre en oeuvre les enseignements de l'invention.

La figure 2 montre la manière dont est analysée, selon l'invention, l'évolution du rythme cardiaque de manière à déclencher et piloter la décroissance du rythme stimulé.

La figure 3 est l'organigramme général de mise en oeuvre de l'invention.

Les figures 4 et 5 illustrent le détail des sous-ensembles référencés respectivement A et B sur la figure 3.

La figure 6 est un tableau de correspondance explicitant la fonction liant la valeur limite du compteur et l'intervalle d'échappement calculé par l'(les) algorithme(s).

La figure 7 est un tableau de correspondance explicitant la fonction liant la valeur du compteur et le taux de décroissance.

La figure 8 illustre les corrélations entre périodes de stimulation, valeur de comptage et taux de décélération du rythme cardiaque.

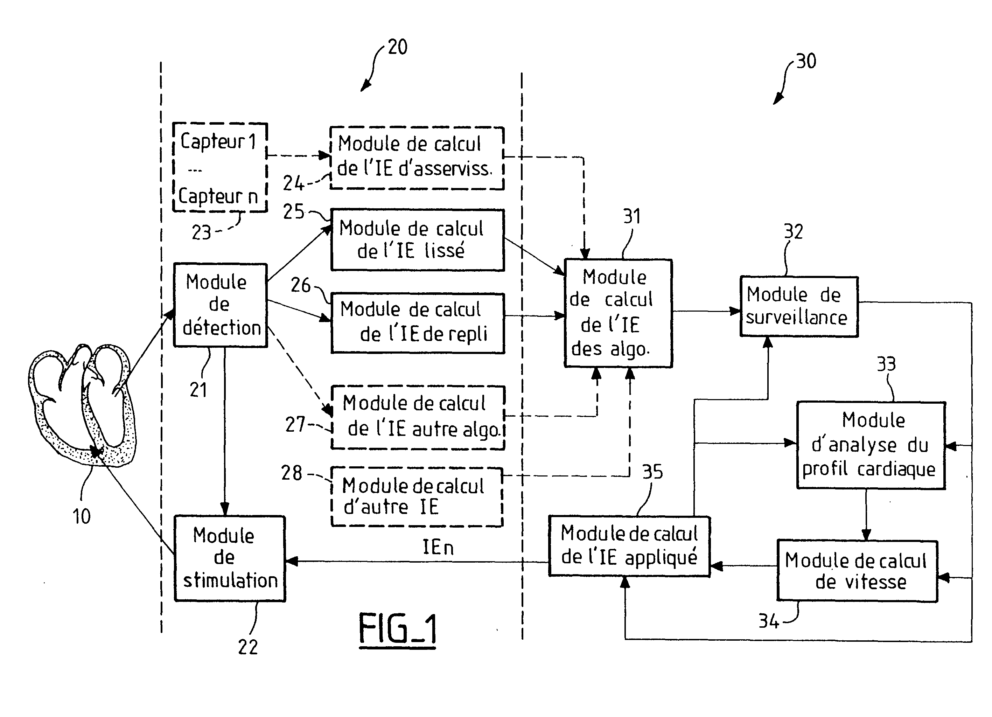

La figure 1 illustre de façon synthétique, sous forme de schéma par blocs, un stimulateur cardiaque susceptible de mettre en oeuvre les enseignements de l'invention. Le stimulateur a été représenté sous forme de deux blocs 20 et 30, composés chacun d'une pluralité de modules interdépendants ; le bloc 20 correspond aux circuits classiques d'un stimulateur, les modules présents de façon facultative étant illustrés en tiretés, et le bloc 30 correspond à la partie de calcul, plus spécifique de la présente invention.

Le coeur 10 du patient produit un rythme spontané (rythme ventriculaire et/ou auriculaire, selon qu'il s'agit d'un stimulateur simple ou multichambre), qui est appliqué à un module de détection 21 du stimulateur. Le dispositif comporte également un module de stimulation 22 fonctionnant à partir d'informations élaborées par le bloc de calcul 30, ou inhibé sur commande du module de détection 21.

Le bloc 20 comporte également — mais de manière facultative — un ou plusieurs capteurs 23 tel qu'un capteur de mesure de la ventilation-minute, de la saturation d'oxygène dans le sang, de la température, de l'accélération, etc., de manière en elle-même connue. Dans ce cas, il est également prévu un module 24 de calcul de l'intervalle d'échappement d'asservissement à partir de l'information recueillie par le ou les capteurs, ce paramètre étant transmis au bloc de calcul 30.

Le bloc 20 comporte également un module 25 de calcul de l'intervalle d'échappement lissé, utilisant l'information du module de détection 21 de la manière décrite par exemple dans le EP-A-0 488 841 (Ela Médical) précité, afin de prévenir les arrêts paroxystiques du rythme cardiaque. Il est également prévu un module 26 de calcul de l'intervalle d'échappement de repli, utilisant l'information du module de détection 21, par exemple comme décrit dans le EP-A-0 488 840 (Ela Médical) précité, pour protéger le ventricule des tachycardies auriculaires dans le cas d'un stimulateur double chambre.

Le bloc 20 peut enfin comprendre un ou plusieurs modules 27, 28 de calcul de l'intervalle d'échappement sur la base d'autres algorithmes encore, utilisant (module 27) ou non (module 28) les informations délivrées par le module de détection 21.

Le bloc 30, qui assure le pilotage du module de stimulation 22 du bloc 20, comporte un module 31 de calcul de l'intervalle d'échappement à partir des données produites par les divers modules 25 à 28. Il détermine un intervalle d'échappement final combinant les divers algorithmes, et désigné ci-après "IEalgo", de manière à satisfaire au mieux la physiologie et la protection du patient.

Cette valeur de IEalgo est appliquée à un module de surveillance 32 discriminant, de la manière que l'on indiquera par la suite, les phases de croissance et de décroissance du rythme cardiaque. Ce module de surveillance 32 pilote un module 33 d'analyse du profil cardiaque, quantifiant les croissances et les décroissances du rythme cardiaque, et un module de calcul de vitesse 34 déterminant le taux de décroissance de la fréquence cardiaque, que l'on appellera par la suite "TauxDec", correspondant à l'accroissement de l'intervalle d'échappement le mieux adapté au patient en fonction de son rythme cardiaque antérieur ; ce taux de décroissance est calculé et mis à jour, de la manière indiquée ci-dessous, pendant les phases de croissance du rythme cardiaque, et il est utilisé pendant les phases de décroissance du rythme cardiaque détectées par le module de surveillance 32.

Le bloc 30 comporte enfin un module 35 de calcul de l'intervalle d'échappement appliqué au bloc 20, déterminant l'intervalle d'échappement du cycle en cours, au terme duquel une impulsion de stimulation est délivrée par le module de stimulation 22 si aucune détection d'une activité spontanée n'est signalée par le module de détection 21 (dans le cas contraire, la stimulation est inhibée).

On va maintenant décrire la manière dont le bloc 30 assure le suivi de l'évolution du rythme cardiaque et pilote la décroissance de la fréquence des impulsions de stimulation.

On utilisera à cet effet les définitions ci-dessous, étant remarqué que l'on fera indifféremment référence soit au rythme ou fréquence cardiaque $\underline{f}$, exprimé en minute$^{-1}$ ou cpm (cycles par minute), soit à l'intervalle d'échappement IE, exprimé en millisecondes, une fréquence croissante correspondant à un intervalle décroissant, et réciproquement.

**Pn-1** : Période cardiaque précédente. Selon les formes de mise en oeuvre, cette période peut être une moyenne de $\underline{x}$ dernières périodes cardiaques ou la période cardiaque précédente proprement dite.

**IEalgo** (précité) : Intervalle d'échappement calculé par un ou plusieurs algorithmes capables de proposer un intervalle inférieur ou égal à la période de base. Par exemple, cet intervalle peut être un intervalle d'asservissement ou un intervalle lissé. Cette valeur d'intervalle concerne la période cardiaque suivant Pn-1, soit Pn.

**IEn** : Intervalle d'échappement calculé par l'invention. Cet intervalle concerne la période cardiaque Pn.

**IEbase** : Intervalle de stimulation de base. Cette valeur est en général fixée par le médecin et est utilisée pour les algorithmes concernés du stimulateur cardiaque.

**IEmin** : Intervalle minimal de stimulation. Cette valeur est en général fixée par le médecin.

**IEseuil** : Valeur d'intervalle d'échappement qui sert à discriminer deux plages de périodes cardiaques différentes et autorise ainsi deux comportements différents de l'algorithme selon la valeur de Pn-1 par rapport à ce seuil.

Dans le présent exemple, IEseuil est arbitrairement fixé à (IEbase + IEmin)/2, mais dans d'autres formes de mise en oeuvre de l'invention, on peut supprimer cette notion d'intervalle seuil ou, au contraire, définir non pas un mais plusieurs intervalles seuils.

**TauxDec (précité)** : Le lissage dynamique pendant la décroissance du rythme cardiaque se fait en choisissant, pour chaque période de décroissance du rythme, un taux de décroissance du rythme adapté au rythme cardiaque antérieur du patient. L'algorithme calcule ce taux de décroissance. Dans le présent exemple, les taux de décroissance sont calculés en nombre de millisecondes (ms) par cycle cardiaque (cc).

**Cpt** : Compteur qui sert à quantifier le profil cardiaque du patient. Sa valeur courante augmente quand le rythme cardiaque du patient s'accélère et diminue quand le rythme cardiaque du patient décélère. C'est la valeur numérique du compteur qui détermine le choix du taux de décélération TauxDec. Dans le présent exemple, Cpt évolue entre 0 et 255.

**SeuilCpt :** Valeur limite du compteur pour une plage de périodes cardiaques donnée.

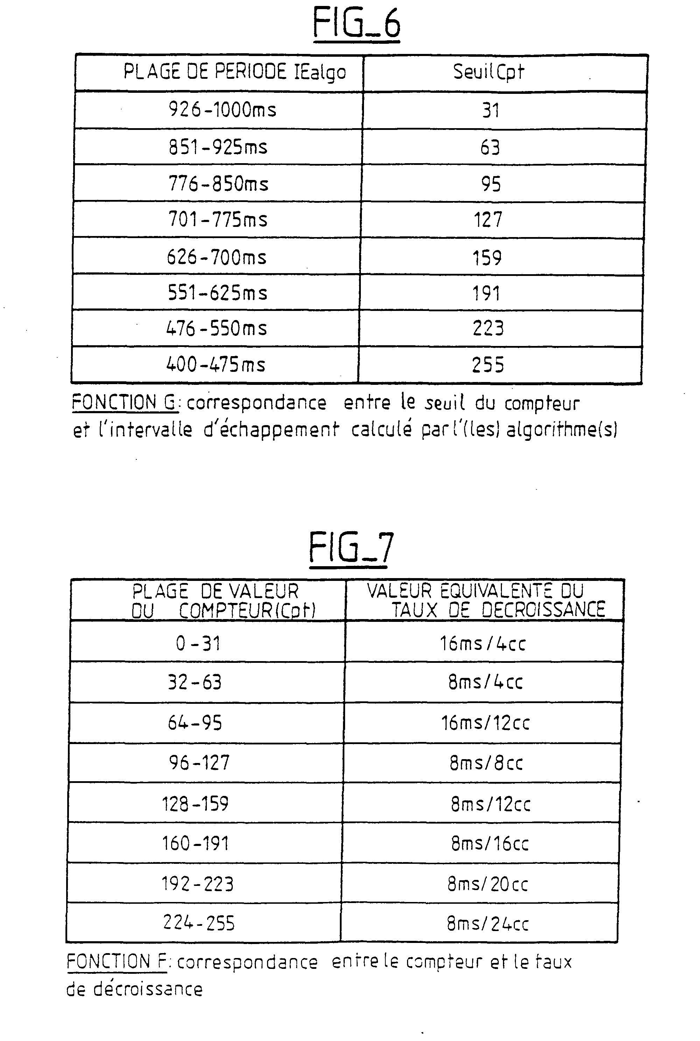

**G(IEalgo)** : Fonction qui détermine la valeur de SeuilCpt en fonction de IEalgo. Cette fonction (explicitée figure 6) est en fait une table de correspondance qui attribue une limite de compteur à chaque valeur de IEalgo. Dans le présent mode de réalisation, l'étendue entre IEbase et IEMin est divisée en huit plages, à chaque plage correspondant une valeur SeuilCpt donnée.

**Macc** et **Pacc** : Variables définissant la façon dont Cpt s'incrémente au cours du temps. Pacc est un pas additif, Macc est un multiplicateur que l'on applique sur ce pas. Dans le présent exemple, Pacc = 1 et Macc = 3.

**Mdec** et **Pdec** : Variables définissant la façon dont Cpt se décrémente au cours du temps. Pdec est un pas soustractif, Mdec est un multiplicateur que l'on applique sur ce pas. Dans le présent exemple, Pdec = 1 et Mdec = 3.

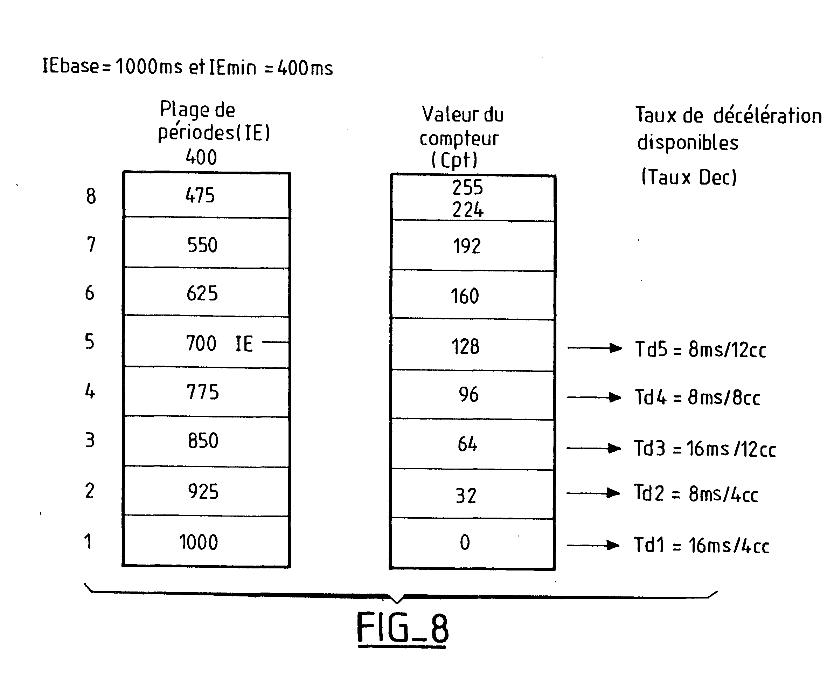

**F(Cpt) :** Fonction qui détermine la valeur de TauxDec en fonction de la valeur de Cpt. Cette fonction (également explicitée figure 7) consiste en fait en une table de correspondance attribuant à chaque valeur de Cpt une valeur de TauxDec. Dans le présent exemple, la table divise les 256 valeurs de Cpt en huit plages de 32 valeurs. A chaque plage correspond une valeur de TauxDec. Par exemple, pour Cpt entre 0 et 31, TauxDec est de 4 ms/cc, correspondant à la décroissance la plus rapide. 4

**Ind** : Indicateur binaire qui sert à déterminer si le rythme cardiaque est croissant ou décroissant. Il est calculé et utilisé à l'intérieur de l'algorithme de l'invention et peut donc prendre deux valeurs : croissance ou décroissance.

**CtrAcc** et **nbSeuilAcc** : Valeurs permettant de s'affranchir des variations non significatives des accélérations du rythme cardiaque stimulé. CtrAcc évolue entre 0 et nbSeuilAcc, qui est fixé dans le présent exemple à 4.

**CtrDec** et **nbSeuilDec :** Valeurs permettant de s'affranchir des variations non significatives des décélérations du rythme cardiaque stimulé. CtrDec évolue entre 0 et nbSeuilDec, qui est fixé dans le présent exemple à 4.

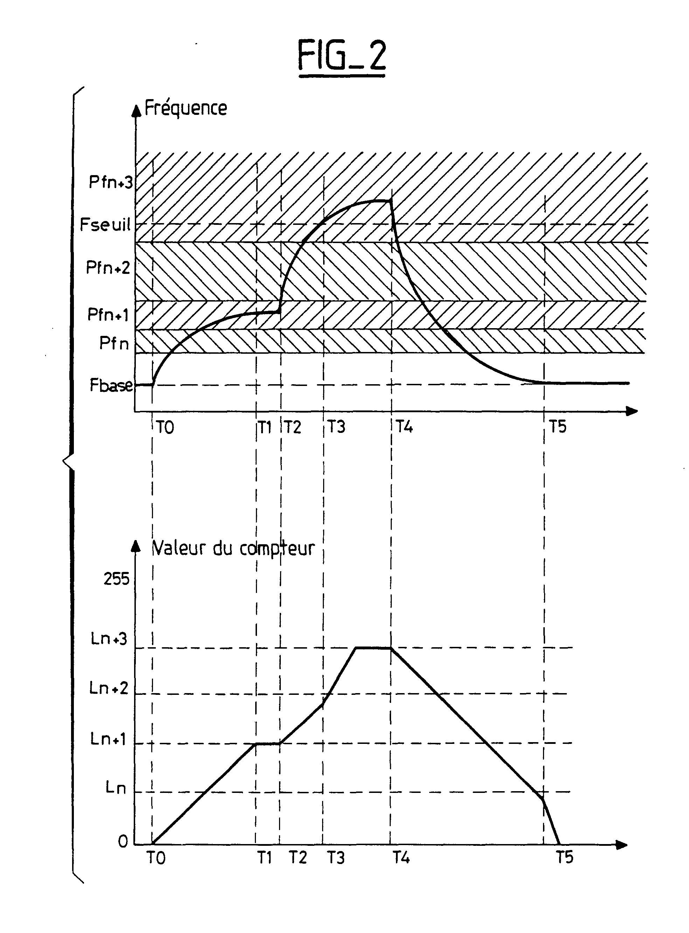

On va maintenant se référer à la figure 2 qui montre, en partie supérieure, un exemple d'évolution de la fréquence cardiaque en réponse à un effort et, en partie inférieure, l'évolution au cours du temps de la variable Cpt définie ci-dessus, c'est-à-dire du contenu du compteur permettant de commander la variation du taux de décélération TauxDec, également définie ci-dessus.

Si l'on considère la courbe illustrant l'évolution de la fréquence cardiaque, entre T0 et T1 on observe une accélération du rythme cardiaque, qui peut être causée par un effort. La fréquence cardiaque se stabilise progressivement (faible variation entre T1 et T2). Entre T2 et T4, on observe une reprise de l'effort avec une accélération corrélative de la fréquence.

Ces accélérations de la fréquence peuvent être sinusales ou provoquées par un algorithme du stimulateur cardiaque, par exemple par un algorithme d'asservissement ou tout autre algorithme susceptible de produire un intervalle de stimulation plus rapide que l'intervalle de base.

Enfin, entre T4 et T5, on observe une décélération du rythme cardiaque, qui peut être aussi bien consécutive à un arrêt sinusal qu'à une commande interne résultant d'un algorithme du stimulateur, par exemple un algorithme d'asservissement.

L'invention a pour objet de piloter cette phase de décélération de la façon la plus physiologique possible, c'est-à-dire en évitant une décélération trop rapide si l'effort antérieur était un effort violent et, inversement, une décélération trop lente après un effort bref, ne nécessitant pas de récupération longue.

On a porté en ordonnée la fréquence Fbase correspondant à l'intervalle de stimulation de base IEbase, ainsi que la fréquence Fseuil correspondant à l'intervalle seuil IEseuil utilisé pour discriminer des plages de rythmes cardiaques différents, croissants ou décroissants.

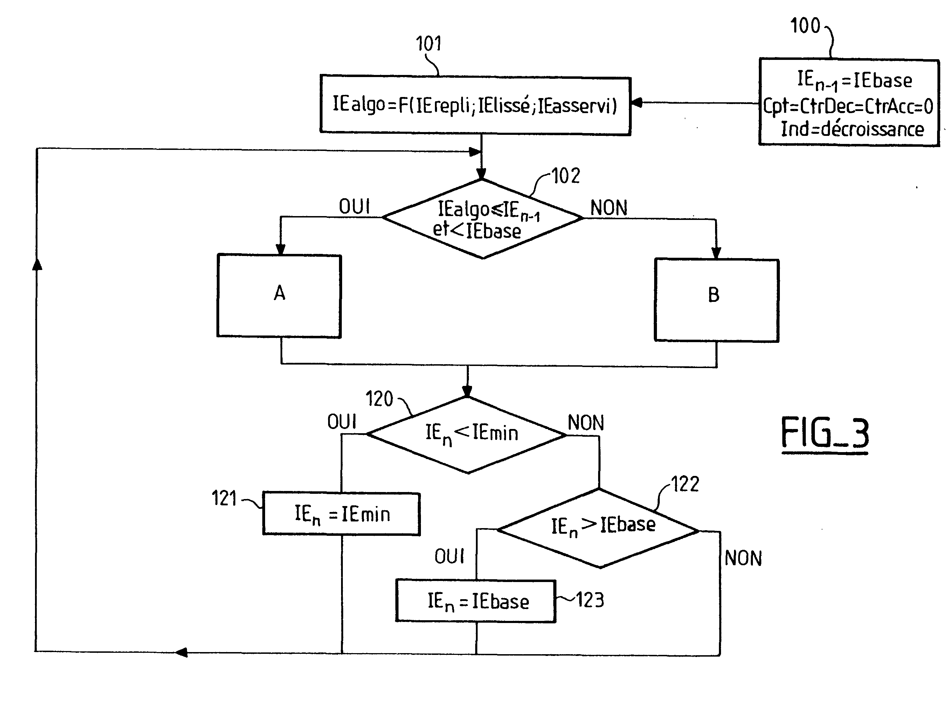

Sur la partie inférieure de la figure 2, on a représenté l'évolution du compteur (variable Cpt) incrémenté avec l'augmentation du rythme cardiaque, et dont la gestion détaillée sera décrite plus en détail en référence aux figures 3 à 6.

Essentiellement, ce compteur joue un double rôle, intégrateur et saturateur : la fonction intégratrice permet de mémoriser et d'analyser l'évolution passée du profil cardiaque (fonction décrivant l'évolution de la fréquence cardiaque en fonction du temps) et la fonction saturatrice permet, par le jeu de seuils et de paliers que l'on explicitera en détail plus loin, de maintenir les effets du pilotage de la décélération dans des limites compatibles avec la physiologie du patient.

EP 0 745 410 B1

Avec l'exemple de profil cardiaque de la figure 1, le compteur évolue de la manière suivante.

Avant T0, le patient est au repos et sa fréquence cardiaque est la fréquence de base. Le compteur reste à une valeur initiale qui est zéro.

Entre T0 et T1, la fréquence cardiaque du patient augmente, tout en restant inférieure à la valeur de seuil Fseuil. La valeur du compteur augmente régulièrement, du pas Pacc (Macc = 1), tout au long de cet effort. À chaque nouvelle valeur du compteur, la valeur du taux de décélération TauxDec est actualisée.

À T1, le compteur atteint la valeur limite du compteur qui correspond à la valeur actuelle de la fréquence cardiaque. Cette valeur limite est calculée avec une table de correspondance telle que celle qui sera décrite à propos de la figure 6. Ainsi, pour une fréquence comprise dans la plage Pfn+1 (figure 2), la limite supérieure du compteur sera Ln+1. Le compteur restera à cette valeur Ln+1 jusqu'à T2.

Entre T2 et T3, la fréquence cardiaque reprend son accélération. La valeur limite du compteur change puisque la fréquence sort de la plage Pfn+1 et le compteur continue d'augmenter régulièrement, du pas Pacc (Macc = 1).

En T3, la fréquence cardiaque devient supérieure au seuil Fseuil. Le compteur augmente alors tous les cycles cardiaques du pas Pacc multiplié par un multiplicateur Macc, jusqu'à atteindre sa nouvelle valeur limite Ln+3. Il se stabilise à cette valeur jusqu'à T4, car la fréquence reste dans la plage Pfn+3.

En T4, une décroissance du rythme cardiaque est détectée. C'est l'algorithme de l'invention qui gère maintenant le calcul d'un intervalle de stimulation décroissant. La valeur du compteur étant Ln+3 au moment de la décroissance, le taux de décroissance (TauxDec) choisi pour la décroissance du rythme cardiaque après T4 est le taux de décroissance correspondant à la valeur Ln+3 du compteur dans la table de correspondance présentée à la figure 7. Pendant la décroissance du rythme cardiaque, le compteur se décrémente tous les cycles cardiaques du pas unitaire Pdec.

En T5, la fréquence cardiaque a atteint sa valeur de base. Le compteur se décrémente alors plus rapidement ; il se décrémente du pas unitaire Pdec multiplié par un multiplicateur Mdec tous les cycles cardiaques, jusqu'à atteindre sa valeur initiale, zéro.

On va maintenant décrire l'algorithme détaillé de gestion du compteur, en référence aux figures 3 à 5.

Le programme débute en 100 lors de sa mise en route.

IEn-1 est initialisé à IEbase, Ind est initialisé à décroissance, CtrAcc et CtrDec sont initialisés à zéro, Cpt est initialisé à zéro et TauxDec est initialisé à sa valeur la plus lente.

L'algorithme détermine d'abord la valeur de IEalgo en 101. Cette valeur est choisie parmi les différents intervalles d'échappement proposés par les différents algorithmes. Dans un exemple de stimulateur cardiaque possédant une fonction d'asservissement et une fonction de lissage du rythme, IEalgo sera déterminé par l'équation :

$$IEalgo = fonction (interv. \text{ lissé } ; interv. \text{ de repli } ; interv. \text{ d'asserv}^t)$$

Plus précisément, lorsque le stimulateur est en phase d'association auriculo-ventriculaire, l'intervalle choisi sera le plus petit entre l'intervalle lissé et l'intervalle d'asservissement. En phase de repli (c'est-à-dire en phase de dissociation auriculo-ventriculaire), l'intervalle d'échappement de repli sera choisi en priorité.

Dans la première phase de l'algorithme, en 102, le programme détermine si le rythme cardiaque est croissant ou décroissant. Cette tâche ainsi que le reste de l'algorithme est effectuée périodiquement : dans cette application, la périodicité du calcul est de quatre cycles cardiaques mais dans d'autres modes de réalisation de l'invention, elle peut être d'un cycle ou, de manière plus générale, de n cycles cardiaques. Pour déterminer si le rythme cardiaque est croissant ou non, l'algorithme compare IEalgo et IEn-1 (IEn-1 est l'intervalle d'échappement cardiaque précédent et IEalgo concerne le cycle cardiaque Pn).

Si IEalgo est strictement supérieur à IEn-1 ou supérieur ou égal à IEbase, une décroissance du rythme cardiaque est suspectée. Ce cas sera traité ultérieurement. Si au contraire IEalgo est inférieur ou égal à IEn-1 et inférieur à IEbase, une croissance du rythme cardiaque est suspectée.

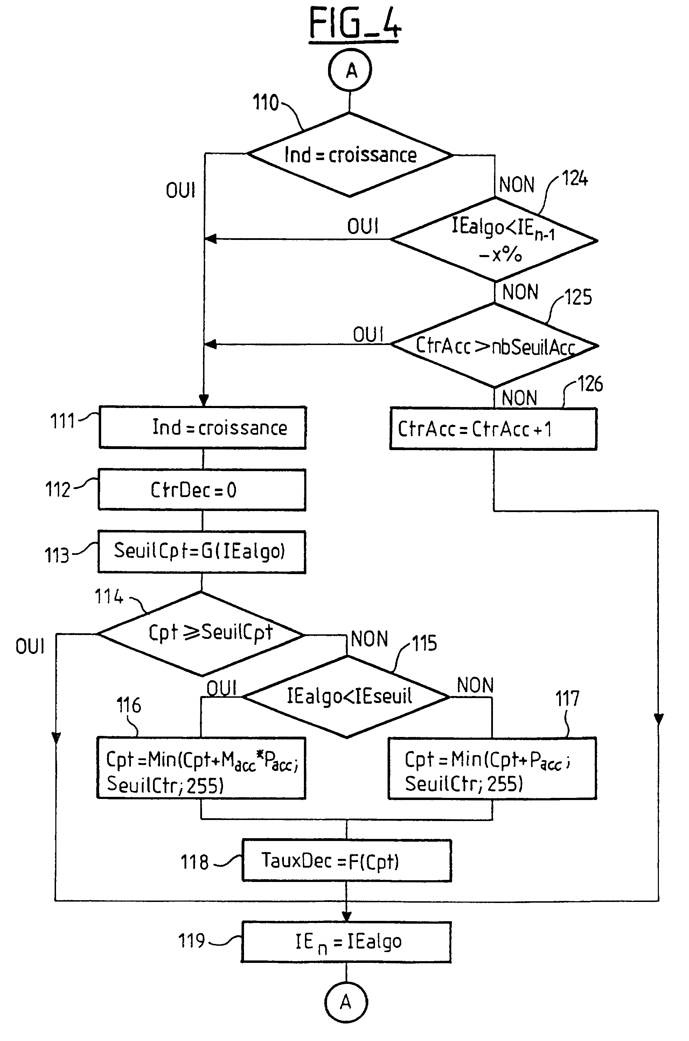

Dans le cas où une croissance du rythme cardiaque est suspectée, l'algorithme passe en 110 (figure 4). Il teste ici l'état de Ind. Si la valeur de Ind est "croissance", cela signifie que la croissance du rythme cardiaque a déjà été confirmée ; l'algorithme force Ind à la valeur "croissance" en 111 et CtrDec à zéro, en 112.

L'algorithme gère maintenant l'évolution de la valeur du compteur. En 113, la limite SeuilCpt est calculée par la fonction G et à partir de la valeur de IEalgo. Cette fonction G est la table de correspondance présentée sur la figure 6.

En 114, la valeur du compteur est comparée à sa limite, calculée en 113. Si Cpt est supérieur ou égal à sa limite, le compteur ne s'incrémente pas et reste constant. L'algorithme passe en 119.

Si au contraire, Cpt est inférieur à sa limite SeuilCpt, le compteur peut s'incrémenter.

La période de croissance se divise en deux phases délimitées par la valeur de IEalgo. C'est l'étape suivante de l'algorithme. En 115, l'algorithme teste la valeur de IEalgo par rapport à la limite IEseuil. Si IEalgo est supérieur à IEseuil, en 117 le compteur prend la valeur minimale entre la valeur du compteur incrémentée du pas Pacc, le SeuilCpt déterminé en 113 et la valeur maximale du compteur, c'est-à-dire 255. Si au contraire, IEalgo est inférieur ou égal à IEseuil, en 116 le compteur prend la valeur minimale entre la valeur du compteur incrémentée du pas Pacc multiplié par Macc, SeuilCpt déterminé en 113 et la valeur maximale du compteur, c'est-à-dire 255.

La valeur du compteur étant actualisée, l'algorithme actualise ensuite la valeur du taux de décroissance à l'aide de la fonction F, en 118. Cette fonction est en fait la table de correspondance qui associe à chaque valeur de Cpt une valeur de TauxDec. Cette table est reproduite figure 7.

La figure 8 explique, à titre d'exemple, les liens de correspondance existant à un moment donné entre la fréquence ou la période cardiaque (IE), la valeur du compteur et le taux de décélération. L'intervalle de périodes existant entre IEbase et IEmin est divisé en huit plages de périodes d'étendue égale. A chacune de ces plages correspond une valeur limite du compteur. Ainsi, pour une période donnée IE se trouvant entre 700 ms et 626 ms, la valeur limite du compteur est 159. Le compteur peut donc prendre une valeur entre 1 et 159. Les 255 valeurs du compteur sont également réparties en huit plages de valeurs d'étendue égale. A chacune de ces plages de valeurs correspond une valeur du taux de décroissance. Ainsi, pour les valeurs du compteur disponibles pour la période IE, selon la valeur atteinte par ce compteur, on peut avoir l'un des cinq taux de décélération se situant entre 16 ms/4 cc et 8 ms/12 cc.

En 119, l'algorithme affecte à IEn une nouvelle valeur (l'invention présentée ne gère que les périodes de décroissance du rythme cardiaque ; pendant les périodes de croissance du rythme cardiaque, l'algorithme affecte à IEn la valeur de IEalgo).

L'algorithme effectue ensuite deux derniers tests pour vérifier que la valeur de IEn ne dépasse pas les bornes inférieure (IEmin) et supérieure (IEbase). En 120, IEn est comparé à IEmin. Si IEn est inférieur à IEmin, on force IEn à IEmin en 121. Si IEn est supérieur ou égal à IEmin, l'algorithme passe au test suivant et compare IEn à IEbase en 122. Si IEn est supérieur à IEbase, IEn est forcé à IEbase en 123. Sinon, IEn est utilisé tel quel. IEn représente l'intervalle de stimulation calculé pour le cycle cardiaque Pn.

Le cas qui vient d'être étudié est le cas où, en 110, Ind est à la valeur "croissance". Si Ind est à "décroissance", c'est-à-dire que la croissance du rythme cardiaque, suspectée en comparant IEalgo et IEn-1 en 102, n'a pas été encore confirmée, l'algorithme se comportera différemment. Avant de faire varier la valeur du compteur, l'algorithme confirme la croissance du rythme.

En 124, l'algorithme réalise le premier test de confirmation de la croissance du rythme cardiaque. Ce dernier concerne la différence entre IEn-1 et IEalgo. Si IEalgo est inférieur à IEn-1 diminué d'une hystérésis (qui est fixée arbitrairement dans cette application à 6,25 %) le rythme cardiaque s'est accéléré significativement et l'algorithme confirme la croissance du rythme. L'algorithme continue à partir de l'étape 111. En revanche, si en 124 IEalgo est supérieur à IEn-1. diminuée de l'hystérésis, la croissance n'est pas confirmée et l'algorithme passe au test suivant, en 125.

Le test réalisé en 125 est une temporisation permettant la confirmation de la croissance du rythme. Il consiste à incrémenter un compteur CtrAcc. Celui-ci est initialisé à zéro dans les périodes de décroissance. Quand la valeur de ce compteur est supérieure à un seuil nbSeuilAcc préalablement défini (fixé ici à 4), cela veut dire que l'algorithme est passé nbSeuilAcc fois consécutives par ce test. La croissance est alors confirmée et l'algorithme se déroule comme précédemment à partir de 111. Si au contraire CtrAcc est inférieur à ce seuil, la croissance n'est toujours pas confirmée. En 126, l'algorithme incrémente la valeur de CtrAcc. Ensuite, la croissance n'étant pas confirmée, l'algorithme n'actualise ni le compteur, ni TauxDec. Puis l'algorithme passe directement en 119 décrit précédemment.

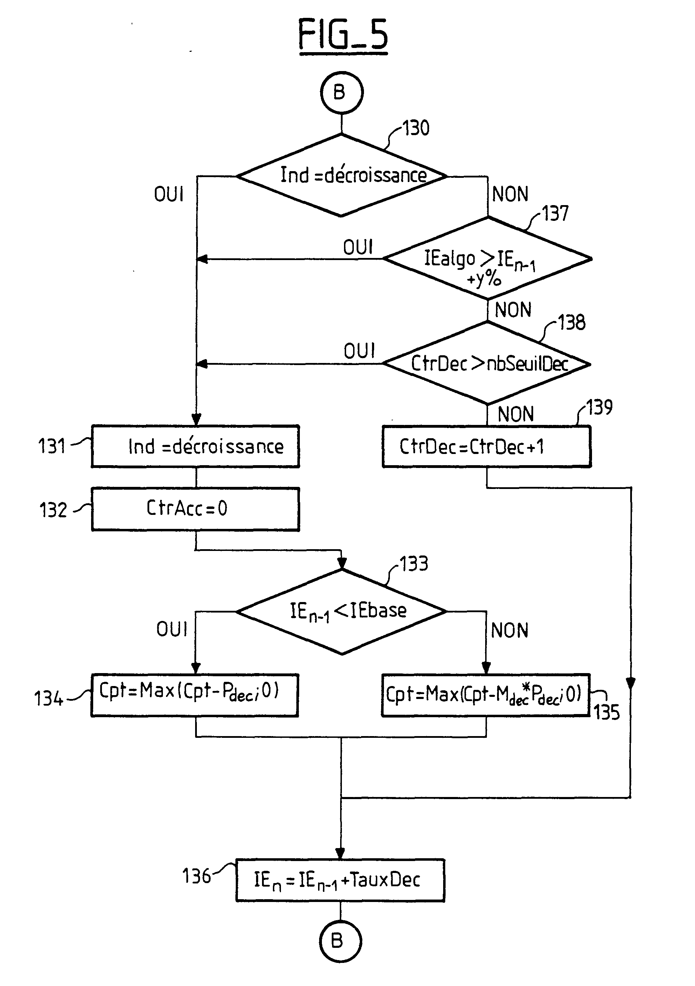

Dans le processus exposé ci-dessus, une croissance du rythme cardiaque avait été suspectée en 102. Si en revanche, en 102, IEalgo est supérieur à IEn-1 ou si IEalgo est supérieur ou égal à IEbase, l'algorithme détecte une décroissance du rythme cardiaque. La valeur de Ind est testée en 130 (figure 5). Si Ind est à décroissance, cela indique que la décroissance du rythme cardiaque a déjà été confirmée. Ind est forcé à "décroissance" en 131 et CtrAcc est forcé à zéro en 132. En 133, l'algorithme teste la valeur de IEn-1. Si IEn-1 est inférieur à IEbase, la décroissance n'est pas terminée et le rythme n'a pas atteint le rythme de base, en 134 le compteur prend la valeur maximale entre la valeur du compteur décrémentée du pas Pdec et zéro. En revanche, si en 133 IEn-1 est supérieur ou égal à IEbase, le rythme cardiaque est retourné au repos, en 135 le compteur prend la valeur maximum entre la valeur du compteur décrémentée du pas Pdec multiplié par Mdec et zéro pour accélérer la diminution de la valeur du compteur. IEn est maintenant affecté en 136. C'est l'invention qui gère la décroissance du rythme cardiaque. Pour cela, l'algorithme dispose de IEn-1, l'intervalle d'échappement cardiaque précédent et de TauxDec, le dernier taux de décroissance actualisé en 118. L'algorithme affecte à IEn la valeur de IEn-1 augmentée de TauxDec.

L'algorithme effectue ensuite en 120,121,122 et 123 les opérations décrites plus haut.

Si en 130, Ind est à la valeur "croissance", l'algorithme effectue un test pour confirmer la décroissance du rythme cardiaque. Le premier test est effectué en 137 avec la composition de IEalgo et de IEn-1. Si IEalgo est supérieur à IEn-1 ajouté d'une hystérésis (fixée ici arbitrairement à 12,5 %), l'algorithme confirme la décroissance du rythme et l'algorithme continue comme précédemment à partir de l'étape 131. Si IEalgo est inférieur à IEn-1 ajouté de l'hystérésis, l'algorithme passe au test suivant, en 138 (l'hystérésis utilisée pendant la période de décroissance peut être dans d'autres modes de réalisation égale à l'hystérésis utilisée pour confirmer la croissance ; le choix a été fait ici de prendre une hystérésis plus importante pour la décroissance afin de rendre plus sévère et plus sélectif le critère de décroissance).

En 138, l'algorithme teste la valeur de CtrDec qui a été initialisé à zéro pendant les périodes de croissance du rythme cardiaque. Ce compteur évolue entre zéro et nbSeuilDec. Si, en 138, CtrDec est supérieur à nbSeuilDec, la décroissance est confirmée et l'algorithme continue à partir de l'étape 131. Si CtrDec est encore inférieur à nbSeuilDec, l'algorithme incrémente la valeur de CtrDec, en 139. Puis, sans réactualiser le compteur, l'algorithme affecte en IEn la valeur de IEn-1 ajouté de TauxDec, en 136.

## Revendications

1.  Un dispositif médical implantable actif, notamment un stimulateur cardiaque ou défibrillateur, comportant :

    — des moyens (21,22,35) pour détecter le rythme cardiaque instantané, spontané ou stimulé, du porteur du dispositif,
    — des moyens (22) pour délivrer des impulsions de stimulation avec un intervalle d'échappement (IEn) variable, et
    — des moyens (30) pour augmenter progressivement cet intervalle d'échappement en fonction de l'évolution dans le temps du rythme cardiaque recueilli par les moyens de détection antérieurement à l'instant (T4) d'augmentation de l'intervalle d'échappement,

    **caractérisé en ce que** les moyens (30) pour augmenter progressivement l'intervalle d'échappement comprennent des moyens (33) aptes à :

    — mémoriser des informations sur ladite évolution dans le temps du rythme cardiaque recueilli par les moyens de détection, et
    — opérer une intégration de ce rythme cardiaque antérieurement à l'instant d'augmentation de l'intervalle d'échappement.

2.  Le dispositif de la revendication 1, dans lequel les moyens pour augmenter progressivement l'intervalle d'échappement déterminent un taux d'accroissement variable (TauxDec) de l'intervalle d'échappement, ce taux variable étant essentiellement déterminé en fonction desdites informations mémorisées.

3.  Le dispositif de la revendication 1, dans lequel les moyens pour augmenter progressivement l'intervalle d'échappement incrémentent et décrémentent un compteur (Cpt), le taux d'accroissement (TauxDec) appliqué à l'instant d'augmentation de l'intervalle d'échappement étant déterminé en fonction de la valeur atteinte par ce compteur.

4.  Le dispositif de la revendication 3, dans lequel le taux d'accroissement appliqué à l'instant d'augmentation de l'intervalle d'échappement varie en sens inverse de la valeur atteinte par le compteur.

5.  Le dispositif de la revendication 3, dans lequel la valeur du compteur est comparée à une série de plages de valeurs prédéterminées, chaque plage correspondant à une valeur donnée du taux d'accroissement appliqué.

6.  Le dispositif de la revendication 3, dans lequel la variation autorisée du compteur est limitée entre une borne minimale et une borne maximale, la borne maximale (SeuilCpt) étant une borne variable fonction d'une valeur calculée d'intervalle d'échappement (IEalgo).

7.  Le dispositif de la revendication 3, dans lequel la vitesse d'incrémentation (Pacc x Macc) du compteur varie en fonction d'une valeur calculée d'intervalle d'échappement (IEalgo).

8.  Le dispositif de la revendication 7, dans lequel on applique à la vitesse d'incrémentation un facteur multiplicateur (Macc) variable en fonction de la valeur calculée d'intervalle d'échappement (IEalgo).

9.  Le dispositif de la revendication 3, dans lequel la vitesse de décrémentation (Pdec x Mdec) du compteur varie en fonction de la valeur de l'intervalle d'échappement déterminée au cycle précédent.

10. Le dispositif de la revendication 9, dans lequel on applique à la vitesse de décrémentation un facteur multiplicateur (Mdec) variable en fonction de la valeur calculée d'intervalle d'échappement (IEalgo).

**EP 0 745 410 B1**

**11.** Le dispositif de la revendication 1, dans lequel, des moyens (32) étant prévus pour déterminer l'état (Ind) croissant ou décroissant, du rythme cardiaque le taux d'accroissement déterminé par les moyens de calcul est appliqué aux moyens pour augmenter progressivement l'intervalle d'échappement lorsqu'un état décroissant est détecté, et maintenu à la valeur ainsi appliquée tant que que cet état ne se modifie pas.

## Claims

**1.** An active implantable medical device, in particular a heart stimulator or defribillator, the device comprising:

· means (21, 22, 33) for detecting the instantaneous, spontaneous, or stimulated heart rate of the bearer of the device;
means (22) for delivering stimulation pulses with a variable escapement interval (IEn); and
· means (30) for progressively increasing said escapement interval as a function of variation over time in the heart rate picked up by the detection means prior to the escapement interval increase instant (T4),
the device being **characterized in that** the means (30) for progressively increasing the escapement interval comprise means (33) suitable for:

· storing information concerning said variation over time in the heart rate picked up by the detection means; and
· integrating said heart rate prior to the escapement interval increase instant.

**2.** The device of claim 1, in which the means for progressively increasing the escapement interval determine a variable rate of increase (TauxDec) for the escapement interval, said variable rate being determined essentially as a function of said stored information.

**3.** The device of claim 1, in which the means for progressively increasing the escapement interval increment and decrement a counter (Cpt), the rate of increase (TauxDec) applied at the escapement interval increase instant being determined as a function of the value reached by the counter.

**4.** The device of claim 3, in which the increase rate applied at the escapement interval increase instant varies inversely with the value reached by the counter.

**5.** The device of claim 3, in which the value of the counter is compared with a series of predetermined ranges of values, each range corresponding to a given value of the applied increase rate.

**6.** The device of claim 3, in which the allowed variation of the counter is restricted between a minimum value and a maximum value, the maximum value (SeuilCpt) being a value that is variable as a function of a value calculated for the escapement interval (IEalgo).

**7.** The device of claim 3, in which the incrementation speed (Pacc × Macc) of the counter varies as a function of a value calculated for the escapement interval (IEalgo).

**8.** The device of claim 7, in which the incrementation speed has a multiplication factor (Macc) applied thereto which is variable as a function of the value calculated for the escapement interval (IEalgo).

**9.** The device of claim 3, in which the decrementation speed (Pdec × Mdec) of the counter varies as a function of the value of the escapement interval determined during the preceding cycle.

**10.** The device of claim 9, in which the decrementation speed has a multiplicative factor (Mdec) applied thereto that is variable as a function of the calculated value for the escapement interval (IEalgo).

**11.** The device of claim 1, in which, for means (32) being provided to determine the increasing or decreasing state (Ind) of the heart rate, the rate of increase determined by the calculation means is applied to the means for progressively increasing the escapement interval when a decrease state is detected, and is maintained at the value as applied in this way so long as said state does not change.

8

**Patentansprüche**

1. Implantierbare aktive medizinische Vorrichtung, insbesondere Herzschrittmacher oder Defibrilator, aufweisend:

   - Mittel zum Detektieren des momentanen, spontanen oder stimulierten Herzrhythmus (21, 22, 39) des Trägers der Vorrichtung,
   - Mittel (22) zum Bereitstellen von Stimulationsimpulsen mit einem variablen Abfallintervall (IEn), und
   - Mittel (30) zum progressiven Erhöhen des Abfallintervalls in Abhängigkeit des Zeitverlaufs des Herzrhythmus, der durch die Detektionsmittel vor dem Augenblick (T4) der Erhöhung des Abfallintervalls aufgenommen worden ist,

   **dadurch gekennzeichnet, dass** die Mittel (30) zum progressiven Erhöhen des Abfallsintervalls Mittel (33) aufweisen, die geeignet sind zum:

   - Speichern von Informationen über den Zeitverlauf des durch die Detektionsmittel empfangenen Herzrhythmus, und
   - Durchführen einer Integration des Herzrhythmus vor dem Augenblick der Erhöhung des Abfallintervalls.

2. Vorrichtung gemäß Anspruch 1, bei welcher die Mittel zum progressiven Erhöhen des Abfallintervalls einen variablen Wachstumswert (TauxDec) des Abfallintervalls bestimmen, wobei der variable Wert im Wesentlichen als Funktion der gespeicherten Informationen bestimmt wird.

3. Vorrichtung gemäß Anspruch 1, bei welcher die Mittel zum progressiven Erhöhen des Abfallintervalls einen Zähler (Cpt) inkrementieren, wobei der Wachstumswert (TauxDec), der in dem Augenblick der Erhöhung des Abfallintervalls angewandt wird, bestimmt wird als Funktion des durch den Zähler erreichten Wertes.

4. Vorrichtung gemäß Anspruch 3, bei welcher der Wachstumswert, der in dem Augenblick der Erhöhung des Abfallintervalls angewendet wird, in entgegengesetzter Richtung zu dem durch den Zähler erreichten Wert variiert.

5. Vorrichtung gemäß Anspruch 3, bei welcher der Wert des Zählers verglichen wird mit einer Reihe von vorbestimmten Wertebereichen, wobei jeder Bereich einem gegebenen Wert des angewandten Wachstumswertes entspricht.

6. Vorrichtung gemäß Anspruch 3, bei welcher die erlaubte Variation des Zählers begrenzt ist zwischen einer minimalen Grenze und einer maximalen Grenze, wobei die maximale Grenze (SeuilCpt) eine Grenze ist, die variabel in Abhängigkeit eines berechneten Werts des Abfallintervalls (IEalgo) ist.

7. Vorrichtung gemäß Anspruch 3, bei welcher die Geschwindigkeit des Inkrementierens (Pacc x Macc) des Zählers variiert in Abhängigkeit eines berechneten Abfallintervalls (IEalgo).

8. Vorrichtung gemäß Anspruch 7, bei welcher auf die Inkrementationsgeschwindigkeit ein Multiplikationsfaktor (Macc) angewandt wird, der variabel ist in Abhängigkeit des berechneten Intervallabfallswertes (IEalgo).

9. Vorrichtung gemäß Anspruch 3, bei welcher die Geschwindigkeit der Dekrementation (Pdec x Mdec) des Zählers variiert in Abhängigkeit des Wertes des Abfallintervalls, welcher im vorherigen Zyklus bestimmt worden ist.

10. Vorrichtung gemäß Anspruch 9, bei welcher auf die Dekrementationsgeschwindigkeit ein Multiplikationsfaktor (Mdec) angewendet wird, der variabel ist als Funktion des berechneten Wertes des Abfallintervalls (IEalgo).

11. Vorrichtung gemäß Anspruch 1, bei welcher, wobei Mittel (32) vorgesehen sind zum Bestimmen des wachsenden oder fallenden Zustands (Ind) des Herzrhythmus, der Wachstumswert, der bestimmt wird durch die Berechnungsmittel, zugeführt wird den Mitteln zum progressiven Erhöhen des Abfallintervalls, wenn ein fallender Zustand detektiert wird, und beim so zugeführten Wert gehalten wird, solange sich der Zustand nicht ändert.

FIG_1

EP 0 745 410 B1

# FIG_2

FIG_3

EP 0 745 410 B1

## FIG_4

(A)

110 — Ind = croissance

OUI → NON

124 — IEalgo<IE$_{n-1}$ −x%  → OUI

NON

125 — CtrAcc>nbSeuilAcc → OUI

NON

111 — Ind = croissance

126 — CtrAcc = CtrAcc +1

112 — CtrDec = 0

113 — SeuilCpt=G(IEalgo)

114 — Cpt ≥ SeuilCpt

OUI / NON

115 — IEalgo<IEseuil

OUI / NON

116 — Cpt =Min(Cpt+M$_{acc}$*P$_{acc}$; SeuilCtr; 255)

117 — Cpt =Min(Cpt+P$_{acc}$; SeuilCtr; 255)

118 — TauxDec =F(Cpt)

119 — IE$_n$ = IEalgo

(A)

# FIG_5

```
        ( B )
          │
         130
    ╱──────────╲
   ╱  Ind =     ╲
OUI ─ décroissance ─ NON
   ╲            ╱
    ╲──────────╱
          │ (NON)
         137
    ╱──────────╲
OUI ─ IEalgo > IE_{n-1} +y% ─
   ╲            ╱
    ╲──────────╱
          │ NON
         138
    ╱──────────╲
OUI ─ CtrDec > nbSeuilDec ─
   ╲            ╱
    ╲──────────╱
          │ NON
```

$IEalgo > IE_{n-1} + y\%$

$CtrDec > nbSeuilDec$

131 — Ind = décroissance

132 — CtrAcc = 0

139 — CtrDec = CtrDec + 1

133 — $IE_{n-1} < IEbase$

OUI / NON

134 — $Cpt = Max(Cpt - P_{dec}; 0)$

135 — $Cpt = Max(Cpt - M_{dec} * P_{dec}; 0)$

136 — $IE_n = IE_{n-1} + TauxDec$

( B )

## FIG_6

| PLAGE DE PERIODE IEalgo | SeuilCpt |
|---|---|
| 926-1000ms | 31 |
| 851-925ms | 63 |
| 776-850ms | 95 |
| 701-775ms | 127 |
| 626-700ms | 159 |
| 551-625ms | 191 |
| 476-550ms | 223 |
| 400-475ms | 255 |

FONCTION G: correspondance entre le seuil du compteur
et l'intervalle d'échappement calculé par l'(les) algorithme(s)

## FIG_7

| PLAGE DE VALEUR DU COMPTEUR(Cpt) | VALEUR EQUIVALENTE DU TAUX DE DECROISSANCE |
|---|---|
| 0-31 | 16ms/4cc |
| 32-63 | 8ms/4cc |
| 64-95 | 16ms/12cc |
| 96-127 | 8ms/8cc |
| 128-159 | 8ms/12cc |
| 160-191 | 8ms/16cc |
| 192-223 | 8ms/20cc |
| 224-255 | 8ms/24cc |

FONCTION F: correspondance entre le compteur et le taux
de décroissance

IEbase = 1000ms et IEmin = 400ms

| Plage de périodes(IE) 400 | Valeur du compteur (Cpt) | Taux de décélération disponibles (Taux Dec) |
|---|---|---|
| 8 — 475 | 255 / 224 | |
| 7 — 550 | 192 | |
| 6 — 625 | 160 | |
| 5 — 700  IE | 128 | Td5 = 8ms/12cc |
| 4 — 775 | 96 | Td4 = 8ms/8cc |
| 3 — 850 | 64 | Td3 = 16ms/12cc |
| 2 — 925 | 32 | Td2 = 8ms/4cc |
| 1 — 1000 | 0 | Td1 = 16ms/4cc |

FIG_8

EP 0 745 410 B1